# EUROPEAN PATENT APPLICATION

(11) **EP 4 803 610 A1**
(43) Date of publication of application: **09.09.2026**
(21) Application number: 24886182.5
(22) Date of filing: 28.10.2024
(51) Int. Cl.: C12N 5/071, C12N 5/079, C12N 5/077, B25J 9/10, G01N 33/50

(54) **BIOHYBRID ROBOT INCLUDING EYE/BRAIN ORGANOID, MOTOR NERVE SPHEROID, AND MUSCLE BUNDLE, AND MANUFACTURING METHOD THEREFOR**

(30) Priority: 30.10.2023 KR 20230146883
(71) Applicant: Sogang University Research & Business Development Foundation, Seoul 04107 (KR)
(72) Inventor: CHOI, Jeong-woo, Seoul 04385 (KR); SHIN, Minkyu, Paju-si, Gyeonggi-do 10920 (KR); LIM, Joung Pyo, Seoul 03767 (KR); AMBROSE, Ashwin Melvin, Seoul 04107 (KR)
(74) Representative: J A Kemp LLP
(86) International application number: PCT/KR2024/016560
(87) International publication number: WO 2025/095508

(57) **Abstract**

The present invention relates to a biohybrid robot comprising an eye/brain organoid, a motor neuron spheroid and a muscle bundle, and a method for fabricating the same, and a versatile biohybrid robot according to the present invention may cause movement of muscle cells by an electrophysiological signal generated from the eye/brain organoid like a human motor system using light stimulation and a neurotransmitter. It is expected to be utilized in disease models that stimulate human signal transduction systems by combining various neurodegenerative disease models, such as Parkinson's and Alzheimer's disease models, with eye tumor models in the future, and be utilized in the fabrication of drug screening platforms using the operation of versatile biohybrid robots composed of biological tissues.

## Description

### [Technical Field]

The present invention was made with the support of the Ministry of Science and ICT under Project No. 1711180504 and Sub-Project No. 2019R1A2C3002300, and the research management institute for the project is the National Research Foundation of Korea, the research program is "Individual Basic Research Program (Ministry of Science and ICT)", the project title is "Biohybrid Robot with Brain Assembloid-based Biomimetic Sensing Functions", the lead institution is Sogang University, and the research period is from March 1, 2023 to February 29, 2024.

In addition, the present invention was made with the support of the Ministry of Science and ICT under Project No. 1711187608 and Sub-Project No. 2022M3H4A1A01005271, and the research management institute for the project is the National Research Foundation of Korea, the research program is "Nanomaterial Technology Development Program", the project title is "Leading Research Center for Diabetes and Metabolic Diseases", the lead institution is Sogang University, and the research period is from January 1, 2023 to December 31, 2023.

In addition, the present invention was made with the support of the Ministry of Science and ICT under Project No. 1711180793 and Sub-Project No. 2022H1D3A2A02093530, and the research management institute for the project is the National Research Foundation of Korea, the research program is "Support for the Expansion of Talent Utilization", the project title is "Ni-TiO2/Photosystem II with Drug Screening Function and Muscle Bundle-Based Nano-Biohybrid Actuator", the lead institution is Sogang University, and the research period is from January 1, 2023 to December 31, 2023.

The present application claims priority to Korean Patent Application No. 10-2023-0146883, filed with the Korean Intellectual Property Office on October 30, 2023, the disclosure of which is incorporated herein by reference.

The present invention relates to a biohybrid robot comprising an eye/brain organoid, a motor neuron spheroid and a muscle bundle, and a method for fabricating the same, and more specifically, to a method for fabricating a versatile biohybrid robot through the connection of an eye/brain organoid derived from human induced pluripotent stem cells (iPSCs), a motor neuron spheroid derived from human neural stem cells (hNSCs) and a muscle bundle derived from three-dimensional skeletal muscle cells (C2C12).

### [Background Art]

In the human motor system, muscle cells contract and relax when electrophysiological signals generated in the brain are transmitted to the muscle cells via motor neurons. However, most bio-robots used in laboratories are based on muscle cells and implement muscle movement through electrical stimulation and motile drugs.

Alternatively, there is a method of implementing muscle movement by external light stimulation through genetic engineering of muscle cells in order to induce muscle cell movement. These methods of inducing muscle movement differ from the muscle movements that occur in actual humans, making it difficult to confirm how brain-induced diseases such as neurodegenerative diseases affect muscle movement, and therefore, there is a need for introducing methods that simulate the human motor system.

One representative method is to introduce brain organoids that are structurally and functionally similar to the brain. Electrophysiological signals generated from brain organoids induce acetylcholine signal generation in connected motor neurons, and the acetylcholine is converted into electrical signals through the neuromuscular junction, thereby inducing muscle contraction.

Prior arts for the fabrication of the aforementioned bio-robots are technologies related to electrical stimulation, the incorporation of nanomaterials, and chemical and drug treatments.

The electrical stimulation technology has the problem that electrodes must be separately fabricated using polymers and graphene to effectively deliver electrical stimulation to muscle cells, and muscle movement must be induced through direct stimulation. The genetic engineering has the positive effect of inducing contraction and relaxation movements in muscle cells through external light stimulation, but it also has the problem that complex steps must be taken for genetic engineering of actual muscle cells. The drug treatment method is relatively simple to use, but it has the disadvantage that various conditions such as the concentration and time of the drug exposed to muscle cells must be controlled.

These methods of inducing muscle movement are different from the actual human motor system in which muscle movement is controlled by signals generated from the brain.

### [Disclosure]

### [Technical Problem]

Accordingly, the present inventors fabricated a biohybrid robot comprising an eye/brain organoid, a motor neuron spheroid and a muscle bundle, and confirmed that a human motor system could be implemented therefrom.

Accordingly, it is an object of the present invention to provide a biohybrid robot comprising:
a conjugate of an eye organoid and a brain organoid;
a motor neuron spheroid connected to the conjugate;
a muscle bundle connected to the motor neuron spheroid; and
a robot structure connected to the muscle bundle.

It is another object of the present invention to provide a method for fabricating a biohybrid robot, comprising the following steps:
a conjugation step of producing a conjugate by conjugating an eye organoid and a brain organoid;
a first connection step of connecting a motor neuron spheroid to the conjugate;
a second connection step of connecting a muscle bundle to the motor neuron spheroid connected to the conjugate; and
a third connection step of connecting a robot structure to the muscle bundle connected to the conjugate.

It is another object of the present invention to provide a method for screening a candidate substance for the treatment of a neurodegenerative disease or eye disease, comprising the following steps:
a motor system preparation step of preparing a biohybrid robot comprising a conjugate of an eye organoid and a brain organoid, a motor neuron spheroid connected to the conjugate, a muscle bundle connected to the motor neuron spheroid and a robot structure connected to the muscle bundle;
a drug contact step of contacting the candidate substance to the biohybrid robot; and
a drug evaluation step of comparing the degree of contraction induced by light stimulation in the muscle bundle after contact with the candidate substance to that in the muscle bundle not contacted with the candidate substance.

Another object of the present invention relates to the use of a biohybrid robot for implementing a human motor system, comprising:
a conjugate of an eye organoid and a brain organoid;
a motor neuron spheroid connected to the conjugate;
a muscle bundle connected to the motor neuron spheroid; and
a robot structure connected to the muscle bundle.

### [Technical Solution]

The present invention relates to a biohybrid robot comprising an eye/brain organoid, a motor neuron spheroid and a muscle bundle, and a method for fabricating the same, and the biohybrid robot according to the present invention may implement a human motor system that can be utilized in screening candidate substances for the treatment of nervous system diseases.

In order to enhance the connection efficiency between the eye/brain organoid and the muscle bundle, the present inventors improved the growth and growth directionality of neurites in the motor neuron spheroid by incorporating hyaluronic acid (HA)-modified gold-nickel-gold nanorods (GNRs) and human umbilical vein endothelial cells (HUVECs) into the motor neuron spheroid.

In order to fix the hyaluronic acid to the surface of the gold-nickel-gold nanorods, the hyaluronic acid was modified with thiol groups using cysteamine and fixed to the surface of the gold-nickel-gold nanorods through S-S bonding. The produced hyaluronic acid-modified gold-nickel-gold nanorods were mixed with the motor neuron spheroid along with the human umbilical vein endothelial cells and an extracellular matrix and used to connect the eye/brain organoid and the muscle bundle, and were fixed on polymeric structures to fabricate the versatile biohybrid robot.

Through a week of co-culture, the eye/brain organoid, the motor neuron spheroid and the muscle bundle were connected on the polymeric structures, and the implementation of the human motor system was confirmed through neurotransmitters or light stimulation of the connected eye/brain organoid. The generation of electrophysiological signals from the eye organoid was confirmed through light stimulation, and the generation of electrophysiological signals from the brain organoid was confirmed through neurotransmitters. The fabricated versatile biohybrid robot demonstrated movement for over a week, and the magnitude of the movement was successfully controlled by neurotransmitters.

Hereinafter, the present invention will be described in more detail.

One aspect of the present invention is a biohybrid robot comprising:
a conjugate of an eye organoid and a brain organoid;
a motor neuron spheroid connected to the conjugate;
a muscle bundle connected to the motor neuron spheroid; and
a robot structure connected to the muscle bundle.

In the present invention, the muscle bundle may have two or more rings.

In the present invention, the robot structure may have two or more connecting portions to be connected to the two or more rings provided on the muscle bundle, and paddles formed by extending from the connecting portions in a direction opposite to the eye organoid and the brain organoid.

The connecting portions may be column-shaped, vertically spaced apart and independently arranged on a single plane for insertion into the ring of the muscle bundle, but are not limited thereto.

Another aspect of the present invention is a method for fabricating a biohybrid robot, comprising the following steps:
a conjugation step of producing a conjugate by conjugating an eye organoid and a brain organoid;
a first connection step of connecting a motor neuron spheroid to the conjugate;
a second connection step of connecting a muscle bundle to the motor neuron spheroid connected to the conjugate; and
a third connection step of connecting a robot structure to the muscle bundle connected to the conjugate.

In the present invention, the conjugation step may be performed by placing the eye organoid and the brain organoid in contact with each other within a hydrogel, but is not limited thereto.

In the present invention, the hydrogel may be an alginate-based sacrificial hydrogel, and the alginate-based hydrogel may be sodium alginate, but is not limited thereto.

In the present invention, the method may further comprise a co-culturing step of co-culturing the eye organoid and the brain organoid after the conjugation step, but is not limited thereto.

In one embodiment of the present invention, the conjugation step finalizes the formation of the conjugate by performing the co-culture step directly in the conjugated state, when the eye organoid and the brain organoid are placed in contact with each other within the alginate-based sacrificial hydrogel and reacted with a potassium chloride (calcium chloride) solution, so that the sodium alginate solution is solidifi, thereby completing the formation of the conjugate by subsequently performing the co-culture step in the conjugated state.

In the present invention, the first connection step may be performed by contacting the conjugate with a hydrogel prepared by mixing one or more selected from the group consisting of gold-nickel-gold nanorods (GNRs), human umbilical vein endothelial cells (HUVECs) and an extracellular matrix with the motor neuron spheroid, and for example, a hydrogel prepared by mixing all of gold-nickel-gold nanorods (GNRs), human umbilical vein endothelial cells (HUVECs) and an extracellular matrix may be used, but is not limited thereto.

The gold-nickel-gold nanorods may be surface-modified with hyaluronic acid (HA), but are not limited thereto.

In the present invention, the muscle bundle may have, but is not limited to, two or more rings.

In the present invention, the robot structure may have two or more connecting portions to be connected to the two or more rings provided on the muscle bundle, and paddles formed by extending from the connecting portions in a direction opposite to the eye organoid and the brain organoid.

The connecting portions may be column-shaped, vertically spaced apart and independently arranged on a single plane for insertion into the ring of the muscle bundle, but are not limited thereto.

Another aspect of the present invention is a method for screening a candidate substance for the treatment of a neurodegenerative disease or eye disease, comprising the following steps:
a motor system preparation step of preparing a biohybrid robot comprising a conjugate of an eye organoid and a brain organoid, a motor neuron spheroid connected to the conjugate, a muscle bundle connected to the motor neuron spheroid and a robot structure connected to the muscle bundle;
a drug contact step of contacting the candidate substance to the biohybrid robot; and
a drug evaluation step of comparing the degree of contraction induced by light stimulation in the muscle bundle after contact with the candidate substance to that in the muscle bundle not contacted with the candidate substance.

In the present invention, the neurodegenerative disease may be selected from the group consisting of Parkinson's disease, Huntington's disease, amyotrophic lateral sclerosis (ALS) and epilepsy, but is not limited thereto.

In the present invention, the eye disease may be selected from the group consisting of macular degeneration, retinitis pigmentosa, Stargardt disease, choroideremia, gyrate-atrophy, dry eye syndrome, eye tumor and eye strain, but is not limited thereto.

### [Advantageous Effects]

The present invention relates to a biohybrid robot comprising an eye/brain organoid, a motor neuron spheroid and a muscle bundle, and a method for fabricating the same, and the versatile biohybrid robot according to the present invention may cause movement of muscle cells by an electrophysiological signal generated from the eye/brain organoid like a human motor system using light stimulation and a neurotransmitter. It is expected to be utilized in disease models that stimulate human signal transduction systems by combining various neurodegenerative disease models, such as Parkinson's and Alzheimer's disease models, with eye tumor models in the future, and to be utilized in the fabrication of drug screening platforms using the operation of versatile biohybrid robots composed of biological tissues.

### [Description of Drawings]

Figure 1a is a schematic diagram showing the production of brain organoids and eye organoids according to one embodiment of the present invention.
Figure 1b is a photograph showing differentiation markers of eye organoids produced according to one embodiment of the present invention, as confirmed by immunostaining.
Figure 1c is a graph showing the degree of differentiation of eye organoids produced according to one embodiment of the present invention, as confirmed by qPCR.
Figure 1d is a fluorescent image showing differentiation markers (S100β, TuJ1, and SOX2) of brain organoids produced according to one embodiment of the present invention, as confirmed by immunostaining.
Figure 1e is a graph showing the degree of differentiation of brain organoids produced according to one embodiment of the present invention, as confirmed by qPCR.
Figure 2a is an optical image showing a conjugate of an eye organoid (RO) and a brain organoid (BO) produced using an alginate-based sacrificial hydrogel according to one embodiment of the present invention.
Figure 2b is an image confirming the conjugation of a brain organoid with an eye organoid containing GFP according to one embodiment of the present invention.
Figure 3a is a schematic diagram showing the control of growth directionality of a motor neuron spheroid produced using hyaluronic acid-modified gold-nickel-gold nanorods and human umbilical vein endothelial cells according to one embodiment of the present invention.
Figure 3b is a photograph showing the surface of gold-nickel-gold nanorods (left) and hyaluronic acid-modified gold-nickel-gold nanorods (right) produced according to one embodiment of the present invention, as confirmed by a transmission electron microscope (TEM).
Figure 3c is a fluorescent image showing the degree of directionality control of a motor neuron spheroid (TuJ1) produced according to one embodiment of the present invention, as confirmed by immunostaining.
Figure 3d is a graph confirming the degree of growth directionality of neural cells in the 0-180° direction in a motor neuron spheroid produced according to one embodiment of the present invention.
Figure 4a is a graph analyzing electrophysiological signals before and after light stimulation of an eye/brain organoid conjugate produced according to one embodiment of the present invention.
Figure 4b is a graph confirming changes in electrophysiological signals using the neurotransmitter glutamate, in an eye/brain organoid conjugate produced according to one embodiment of the present invention.
Figure 5a is a schematic diagram showing the connection of an eye/brain organoid, a motor nerve spheroid and a muscle bundle for fabricating a versatile biohybrid robot according to one embodiment of the present invention.
Figure 5b is a fluorescent image showing the connection of an eye/brain organoid (GFP), a motor neuron spheroid (TuJ1) and a muscle bundle (F-actin) produced according to one embodiment of the present invention, as confirmed by immunostaining.
Figure 5c is an image showing a versatile biohybrid robot fabricated according to one embodiment of the present invention.
Figure 5d is a drawing confirming changes in movement by the neurotransmitter glutamate, in a versatile biohybrid robot fabricated according to one embodiment of the present invention.

### [Best Mode for Carrying Out the Invention]

The present invention relates to a biohybrid robot comprising a conjugate of an eye organoid and a brain organoid, a motor neuron spheroid connected to the conjugate, a muscle bundle connected to the motor neuron spheroid, and a robot structure connected to the muscle bundle.

### [Mode for Carrying out the Invention]

Hereinafter, the present invention will be described in more detail by way of the following examples. However, these examples are only for illustrating the present invention, and the scope of the present invention is not limited to these examples.

Throughout this specification, "%" used to indicate the concentration of a particular substance is (weight/weight)% for solid/solid, (weight/volume)% for solid/liquid, and (volume/volume)% for liquid/liquid, unless otherwise stated.

### Example 1: Production of eye/brain organoids, motor neuron spheroids and muscle bundles.

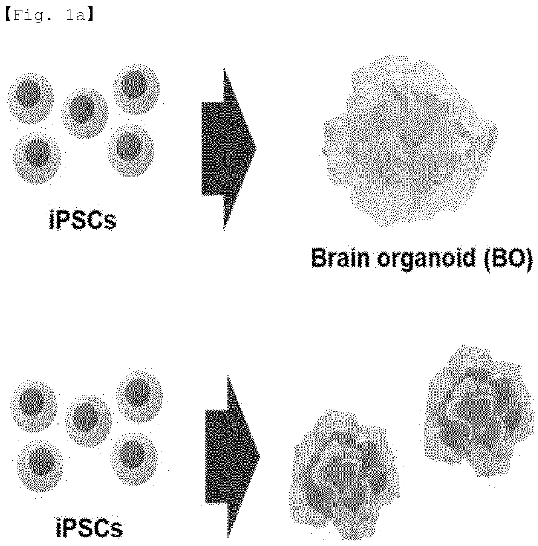

Eye/brain organoids that receive light and generate electrophysiological signals similar to human eyes were produced using human induced pluripotent stem cells (iPSCs) as shown in Figure 1a.

### 1-1. Production of eye organoids

Human induced pluripotent stem cells were formed into eye organoids through three differentiation stages. First, embryonic bodies composed of 1.0 × 10⁴ cells/mL were cultured in DMEM/F12 (1:1) medium containing 1% N2 supplement, 1% minimum essential amino acids (NEAA) and 2 mg/mL heparin. After attachment on a Matrigel-coated plate on day 7 of culture, the culture was continued, and on day 16 of culture, the culture medium was changed to DMEM/F12 (3:1) containing 2% B27 (without vitamin A), 1% NEAA and 1% antibiotic-antimycotic for differentiation into eye cells.

On week 4 of differentiation, the formed horseshoeshaped eye region was isolated using a sharp tungsten needle and then cultured to gradually form 3D eye organoids. For long-term culture, the medium was supplemented with 10% fetal bovine serum, 100 mM taurine and 2 mM GlutaMAX from day 42. Subsequently, the production of eye organoids was confirmed through immunostaining and electrophysiological signal measurements.

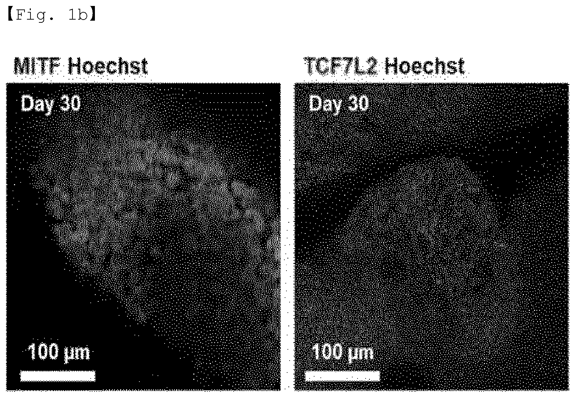

As shown in Figure 1b, MITF (microphthalmia-associated transcription factor), which is a visual cell differentiation marker observed in the intermediate stage of eye organoid formation, was observed.

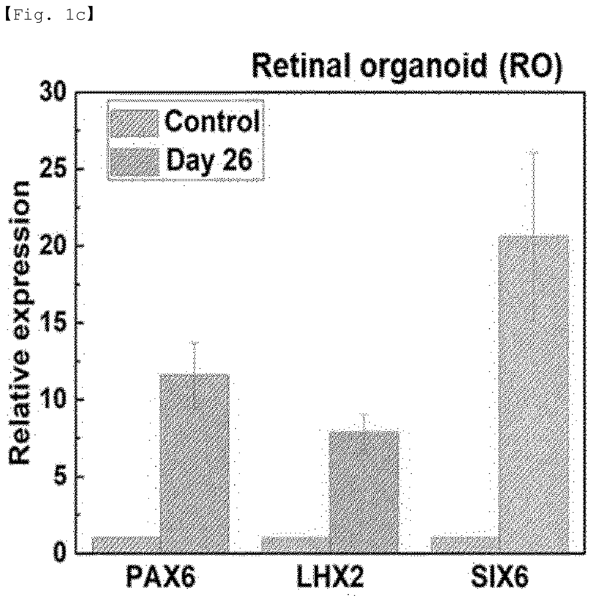

As shown in Figure 1c, quantitative polymerase chain reaction (quantitative PCR; qPCR) measurement results showed that the expression levels of PAX6, LHX2 and SIX6, which are genes related to visual cells, increased with eye organoid differentiation.

### 1-2. Production of brain organoids

Human induced pluripotent stem cells were formed into brain organoids through four differentiation stages. Embryoid bodies composed of 1.0 × 10⁴ cells/mL were cultured for 4 days in StemFit Basic04 containing 50 µM Rho-associated protein kinase (ROCK) inhibitor and 4 ng/mL basic fibroblast growth factor (bFGF). On day 5 of culture, cells were cultured for 5 days in DMEM/F-12 containing 1% N2 supplement, 1% GlutaMAX supplement, 1% NEAA solution and 1 µg/mL heparin. On day 9 of culture, the differentiating brain organoids were embedded using 5 µL of Matrigel and then cultured for 4 days in DMEM/F-12: Neurobasal media (1:1) containing 1% N2 supplement, 1% B-27 supplement without vitamin A, 1% GlutaMAX supplement, 1% NEAA solution, 50 µM 2-mercaptoethanol, and 2.5 µg/mL insulin, 100 µg/mL streptomycin and 100 U/mL penicillin.

Finally, on day 13, the 1% B-27 supplement in the existing media was replaced with a 1% B-27 supplement containing vitamin A, and long-term culture was performed. The structure and function of the produced brain organoids were confirmed through immunostaining and electrophysiological signal measurements.

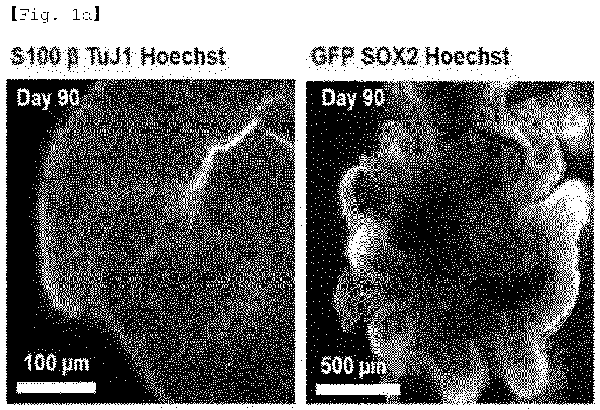

As shown in Figure 1d, the neural cell differentiation markers S100β (S100 calcium-binding protein β), TuJ1 (neuron-specific class III beta-tubulin) and SOX2 (SRY (sex-determining region Y)-box 2) observed during brain organoid formation showed that neural networks and structures were formed inside the brain organoids.

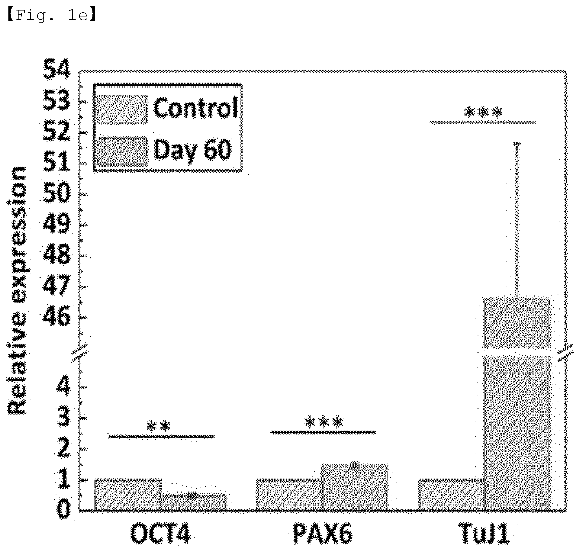

As shown in Figure 1e, quantitative polymerase chain reaction (qPCR) measurement results showed that with brain organoid differentiation, the expression level of OCT4, which is a stem cell marker, decreased and the expression levels of PAX6 and TuJ1, which are genes related to neural cells, increased.

### 1-3. Production of conjugates of eye organoids and brain organoids

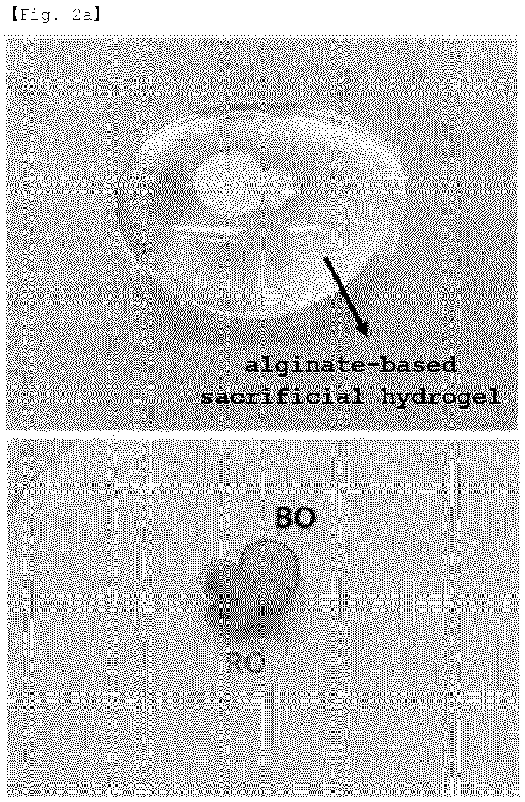

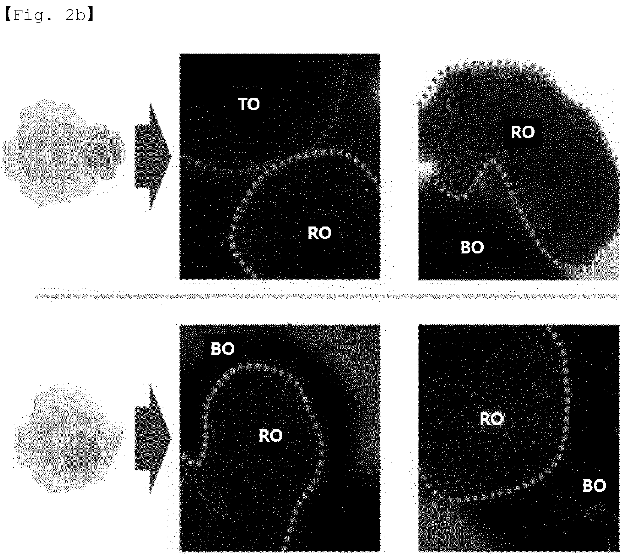

Eye organoids and brain organoids were connected using an alginate-based sacrificial hydrogel. After the brain organoids and the eye organoids were placed in a 1 g/100 ml sodium alginate solution, the retinal organoids were physically conjugated to the brain organoids using tweezers. Thereafter, when a 4 g/100 ml calcium chloride solution is sprayed, the sodium alginate solution solidifies, and the brain organoids and the eye organoids also solidify in a conjugated state. Subsequently, when the conjugates of brain organoids and eye organoids are cultured in co-culture media for 3 days, the solidified sodium alginate naturally decomposes, and well-conjugated conjugates of brain organoids and eye organoids are formed, as shown in Figures 2a and 2b.

### 1-4. Production of motor neuron spheroids

To produce motor neuron spheroids, human neural stem cells (hNSCs) were cultured in KnockOut^{™} DMEM/F-12 containing 20 ng/mL bFGF (basic fibroblast growth factor) and 20 ng/mL EGF. Spheroids were produced using human neural stem cells at 7.0 × 10⁴ cells/well, and after 48 hours, the culture medium was replaced with motor neuron differentiation medium containing 8 ng/mL bFGF, 200 ng/mL sonic hedgehog, 10 ng/mL activin A and 50 µM retinoic acid. On day 20 of differentiation, the existing motor neuron differentiation medium was replaced with a motor neuron differentiation medium containing 10 ng/mL BDNF (brain-derived neurotrophic factor) and 10 ng/mL GDNF (glial cell-derived neurotrophic factor) and cultured for 8 days for maturation of motor neuron spheroids.

### 1-5. Production of muscle bundles

To produce muscle bundles, a structure was fabricated using a stereolithography 3D printer, and then PDMS was poured into the structure to create a mold for a muscle bundle production. Muscle bundles were produced by mixing 350 µL of skeletal muscle cells (C2C12) at 5 × 10⁶ cells/mL, 300 µL of Matrigel, 4 mg/mL fibrinogen and 2 U/mL thrombin, placing 200 µL of the mixture in the mold for a muscle bundle production and allowing gelation for 30 minutes. The produced muscle bundles were able to demonstrate movement after approximately two weeks of differentiation, and DMEM containing 2% horse serum, 1 mg/mL aminocaproic acid, 1 ng/mL insulin growth factor-1 and 1% penicillin/streptomycin was used for differentiation. Differentiation of the produced muscle bundles was confirmed through immunostaining.

### Example 2: Control of the growth directionality of motor neuron spheroids using hyaluronic acid-modified gold-nickel-gold nanorods

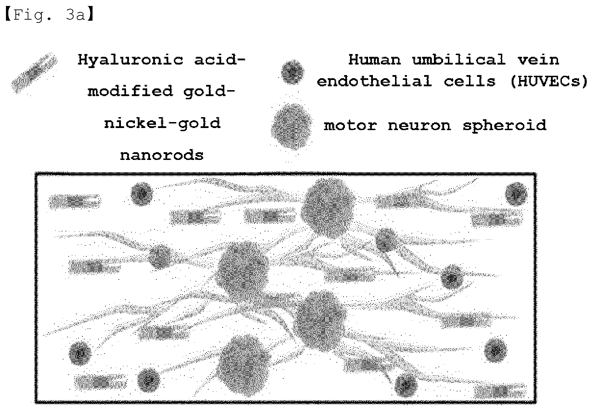

To control the growth directionality of the motor neuron spheroids produced in Examples 1-4 above, gold-nickel-gold nanorods (GNRs) and hyaluronic acid (HA) were used to induce the growth of axons in neural cells, as shown in Figure 3a.

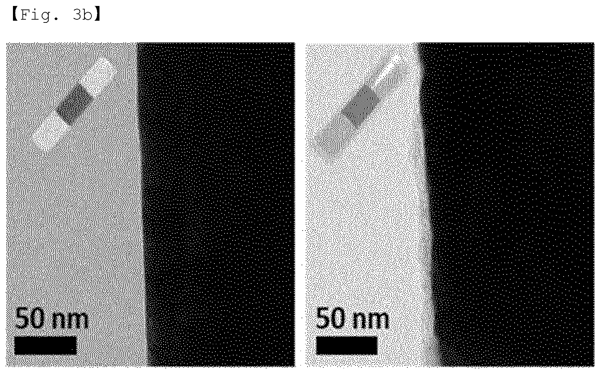

Specifically, to fix hyaluronic acid on the surface of the gold-nickel-gold nanorods, cysteamine was used to produce a thiol group-modified hyaluronic acid, as shown in Figure 3b. To produce hyaluronic acid-modified gold-nickel-gold nanorods, gold-nickel-gold nanorods were first synthesized using a 200 nm porous membrane. For synthesis, silver was physically deposited on the surface of the porous membrane, and then gold, nickel and gold were deposited in that order through electrochemical deposition. The porous membrane and silver used for production were dissolved using 3 M sodium hydroxide and nitric acid, and then the gold-nickel-gold nanorods were recovered and washed using a magnet.

To attach hyaluronic acid to the produced gold-nickel-gold nanorods, 10 mL of 0.1 mg/mL gold-nickel-gold nanorods was mixed with 4 mg of thiol group-attached hyaluronic acid, reacted at room temperature for 24 hours, and then recovered and washed using a magnet. The produced hyaluronic acid-modified gold-nickel-gold nanorods were mixed with motor neuron spheroids, human umbilical vein endothelial cells (HUVECs) and an extracellular matrix to prepare a hybrid hydrogel, which was used to connect eye/brain organoids and motor neuron spheroids. The hybrid hydrogel was prepared by mixing 60 µL of Matrigel, 20 µL of hyaluronic acid-modified gold-nickel-gold nanorods (0.5 mg/mL), 68 µL of 1 × 106 human umbilical vein endothelial cells and 50 µL of fibrinogen (16 mg/mL), and 20 µL of the hybrid hydrogel was used to connect eye/brain organoids and motor neuron spheroids.

To confirm the control of growth directionality of motor neuron spheroids using hyaluronic acid-modified gold-nickel-gold nanorods, three additional groups of motor neuron spheroids [(i) motor neuron spheroids (w/o hyaluronic acid-modified gold-nickel-gold nanorods, human umbilical vein endothelial cells), (ii) motor neuron spheroids (w/o hyaluronic acid-modified gold-nickel-gold nanorods, w/ human umbilical vein endothelial cells), (iii) motor neuron spheroids (w/ hyaluronic acid-modified gold-nickel-gold nanorods, w/ human umbilical vein endothelial cells)] were produced. After approximately 7 days, the neural cell growth and growth directionality of the produced three groups of motor neuron spheroids were confirmed by TuJ1 staining.

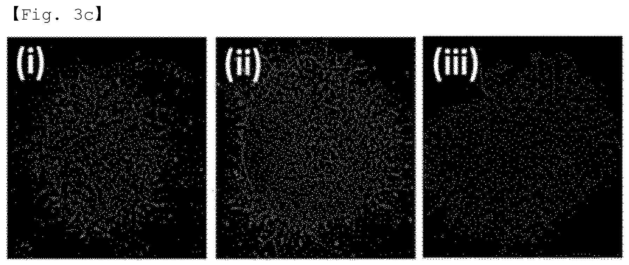

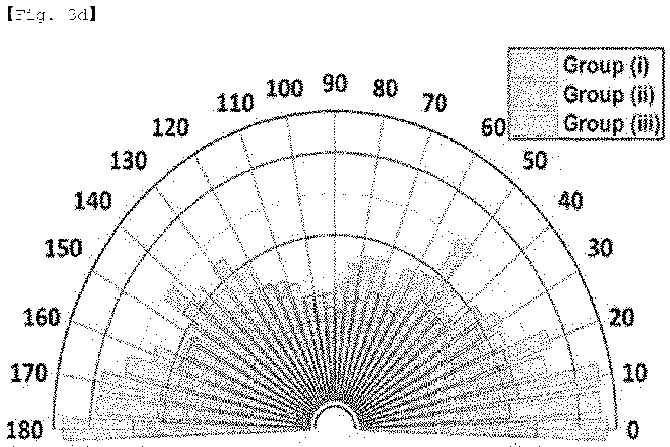

As shown in Figures 3c and 3d, group (iii) showed a 1.34-fold higher directionality of neural cells in the 0-10° direction compared to group (i).

### Example 3: Control of electrophysiological signals in eye/brain organoids through light stimulation and neurotransmitters

The formation of conjugates of brain organoids and eye organoids in Examples 1-3 above was confirmed through signal measurement using a multi-electrode array. Eye/brain organoids were placed on electrodes of the multi-electrode array for signal measurement, and then the generation of electrophysiological signals was measured while light was applied to the eye organoids. In addition, glutamate was added as a neurotransmitter to the co-culture medium used in the multi-electrode array, and electrophysiological signals generated from brain organoids were measured.

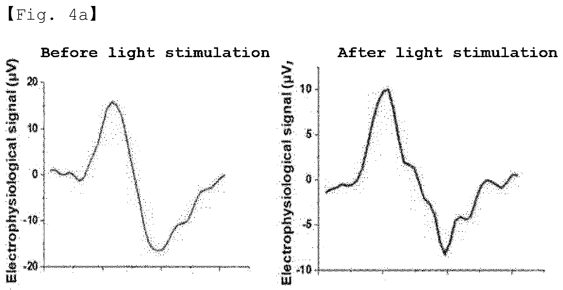

As shown in Figure 4a, when electrophysiological signals were generated by applying light stimulation (98 mW/mm2, 1s) in the visible light range to eye organoids, the electrophysiological signals were transmitted to adjacent brain organoids.

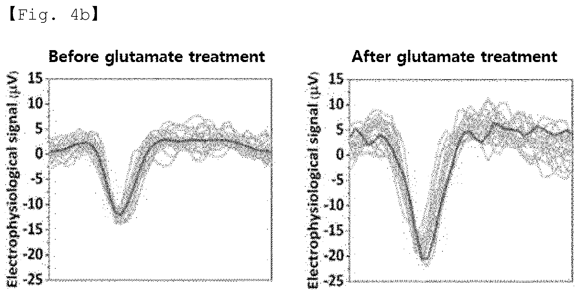

In addition, as shown in Figure 4b, the electrophysiological signals generated by stimulating brain organoids by mixing 100 µM glutamate into the co-culture medium were also transmitted to the brain organoids.

### Example 4: Fabrication and operational verification of versatile biohybrid robots

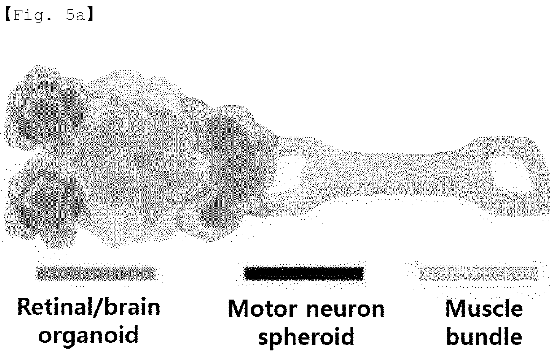

To fabricate a versatile biohybrid robot as shown in Figure 5a, a structure was fabricated using a stereolithography 3D printer, and then Ecoflex:PDMS (5:1) was poured into the structure to fabricate a versatile biohybrid robot structure. Muscle bundles on day 7 of differentiation were attached to the fabricated versatile biohybrid robot structure, and then connected to eye/brain organoids using a hybrid hydrogel containing hyaluronic acid-modified gold-nickel-gold nanorods and motor neuron spheroids.

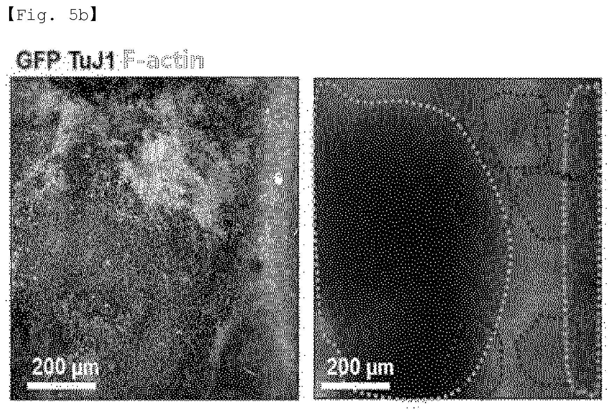

As shown in Figure 5b, the connection of an eye/brain organoid (GFP), a motor neuron spheroid (TuJ1) and a muscle bundle (F-actin) was confirmed by immunostaining from the versatile hybrid robot.

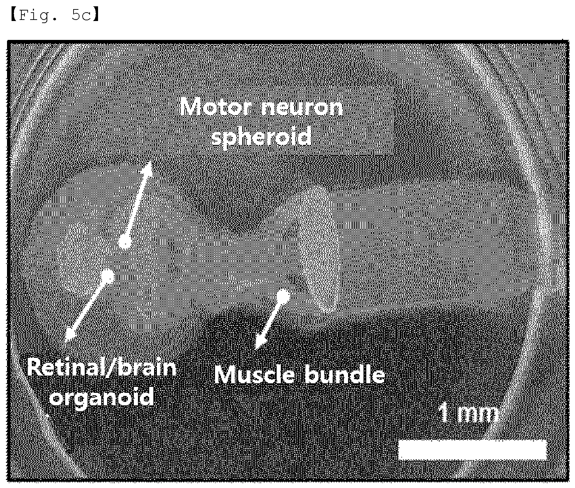

The versatile biohybrid robot as shown in Figure 5c was co-cultured for 7 days in a co-culture media containing a mixture of eye/brain organoid co-culture media and motor neuron spheroid/muscle bundle co-culture media. After 7 days, the eye/brain organoid, the motor neuron spheroid and the muscle bundle were connected, and then the fabricated versatile biohybrid robot moved forward in co-culture media.

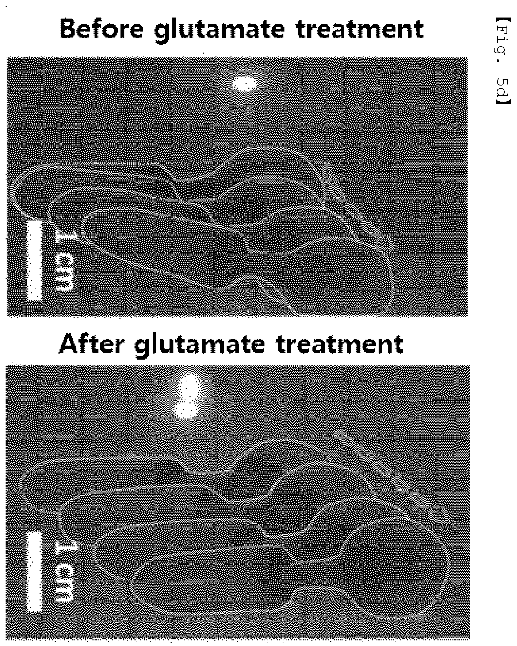

In addition, as shown in Figure 5d, when the brain organoid was stimulated using glutamate, it could be confirmed that the movement improved from 0.27 cm/min before stimulation to 0.52 cm/min after stimulation, and the movement was confirmed to have persisted for over a week.

### [Industrial Availability]

The present invention relates to a biohybrid robot comprising an eye/brain organoid, a motor neuron spheroid and a muscle bundle, and a method for fabricating the same, and more specifically, to a method for fabricating a versatile biohybrid robot through the connection of an eye/brain organoid derived from human induced pluripotent stem cells (iPSCs), a motor neuron spheroid derived from human neural stem cells (hNSCs) and a muscle bundle derived from three-dimensional skeletal muscle cells (C2C12).

## Claims

1. A biohybrid robot comprising:
a conjugate of an eye organoid and a brain organoid;
a motor neuron spheroid connected to the conjugate;
a muscle bundle connected to the motor neuron spheroid; and
a robot structure connected to the muscle bundle.

2. The biohybrid robot according to claim 1, wherein the muscle bundle has two or more rings.

3. The biohybrid robot according to claim 2, wherein the robot structure has two or more connecting portions to be connected to the two or more rings provided on the muscle bundle, and paddles formed by extending from the connecting portions in a direction opposite to the eye organoid and the brain organoid.

4. A method for fabricating a biohybrid robot, comprising the following steps:
a conjugation step of producing a conjugate by conjugating an eye organoid and a brain organoid;
a first connection step of connecting a motor neuron spheroid to the conjugate;
a second connection step of connecting a muscle bundle to the motor neuron spheroid connected to the conjugate; and
a third connection step of connecting a robot structure to the muscle bundle connected to the conjugate.

5. The method according to claim 4, wherein the conjugation step is performed by placing the eye organoid and the brain organoid in contact with each other within a hydrogel.

6. The method according to claim 5, wherein the hydrogel is an alginate-based sacrificial hydrogel.

7. The method according to claim 4, wherein the method further comprises a co-culturing step of co-culturing the eye organoid and the brain organoid after the conjugation step.

8. The method according to claim 4, wherein the first connection step is performed by contacting the conjugate with a hydrogel prepared by mixing one or more selected from the group consisting of gold-nickel-gold nanorods (GNRs), human umbilical vein endothelial cells (HUVECs) and an extracellular matrix with the motor neuron spheroid.

9. The method according to claim 8, wherein the gold-nickel-gold nanorods are surface-modified with hyaluronic acid (HA).

10. The method according to claim 4, wherein the muscle bundle has two or more rings.

11. The method according to claim 10, wherein the robot structure has two or more connecting portions to be connected to the two or more rings provided on the muscle bundle, and paddles formed by extending from the connecting portions in a direction opposite to the eye organoid and the brain organoid.

12. A method for screening a candidate substance for the treatment of a neurodegenerative disease or eye disease, comprising the following steps:
a motor system preparation step of preparing a biohybrid robot comprising a conjugate of an eye organoid and a brain organoid, a motor neuron spheroid connected to the conjugate, a muscle bundle connected to the motor neuron spheroid and a robot structure connected to the muscle bundle;
a drug contact step of contacting the candidate substance to the biohybrid robot; and
a drug evaluation step of comparing the degree of contraction induced by light stimulation in the muscle bundle after contact with the candidate substance to that in the muscle bundle not contacted with the candidate substance.

13. The method according to claim 12, wherein the neurodegenerative disease is selected from the group consisting of Parkinson's disease, Huntington's disease, amyotrophic lateral sclerosis (ALS) and epilepsy.

14. The method according to claim 12, wherein the eye disease is selected from the group consisting of macular degeneration, retinitis pigmentosa, Stargardt disease, choroideremia, gyrate-atrophy, dry eye syndrome, eye tumor and eye strain.
